# EUROPÄISCHE PATENTSCHRIFT

(11) **EP 1 067 110 B1**
(45) Veröffentlichungstag und Bekanntmachung des Hinweises auf die Patenterteilung: **30.07.2003**
(21) Anmeldenummer: 00112849.5
(22) Anmeldetag: 17.06.2000
(51) Int. Cl.: C07C 67/03, C07C 69/013, C07C 69/54

(54) **Verfahren zur Herstellung und Prozessinhibierung von Isobornyl(meth)acrylat**
Process for the preparation and process inhibition of isobornyl(meth)acrylate
Procédé de préparation et d'inhibition de procédé du (méth)acrylate d'isobornyle

(30) Priorität: 05.07.1999 DE 19930721
(43) Veröffentlichungstag der Anmeldung: 10.01.2001
(73) Patentinhaber: Röhm GmbH & Co. KG, 64293 Darmstadt (DE)
(72) Erfinder: Knebel, Joachim, Dr., 64665 Alsbach-Hähnlein (DE); Saal, Doris, 64625 Bensheim (DE)

(56) Entgegenhaltungen:
- EP-A- 0 781 758
- LUCIA HELENA B. BAPTISTELLA ET AL.: "1,8-Diazabicyclo(5.4.0)undec-7-ene as a Mild Deprotective Agent for Acetyl Groups" SYNTHESIS., Nr. 6, 1989, Seiten 436-438, XP002152124 GEORG THIEME VERLAG. STUTTGART., DE ISSN: 0039-7881

## Beschreibung

Die Erfindung betrifft ein Verfahren zu Herstellung und zur Prozeßinhibierung von Isobornyl(meth)acrylat. Isobornyl findet als Monomer breite Anwendung zur Herstellung beispielsweise von Lackbindemitteln.

### Stand der Technik

Die übliche Herstellungsweise von Isobornyl(meth)acrylat besteht darin, daß man Camphen mit Methacrylsäure in Gegenwart eines sauren Katalysators umsetzt.

So beschreibt beispielsweise die DE 44 19 686 (Hoechst AG) ein Verfahren zur Herstellung von Terpenestern durch Reaktion von Camphen und einer niedermolekularen Carbonsäure mit einem sauren Ionenaustauscher als Katalysator. Die Reaktanten werden von unten durch einen säulenförmigen Reaktor geschickt, in dem sich das saure lonenaustauscherbett befindet. Nachteilig bei diesem Verfahren ist allerdings, daß der Katalysator nach einigen Reaktionszyklen an Aktivität verliert. Diesen Aktivitätsverlust kann man durch Temperaturerhöhung versuchen auszugleichen, handelt sich aber den Nachteil der zunehmenden Polymerisation der ungesättigten Carbonsäuren am Katalysator und im Laufe der Zeit die endgültige Deaktivierung des Katalysators ein. Das Verfahren ist daher für großtechnische Anwendungen, die lange Katalysatorstandzeiten erfordern, nicht geeignet.

EP 718 271 (Elf Atochem) beschreibt ebenfalls die Herstellung von Isobornylmethacrylat oder -acrylat durch Umsetzung von Camphen mit Acrylsäure oder (Meth)acrylsäure in Gegenwart eines sauren lonenaustausch-Katalysators. Die auf den ersten Blick elegante lonenaustausch-Katalyse hat aber in der industriellen Praxis den Nachteil, daß der lonenaustauscher beim wiederholten Einsatz an Aktivität verliert, da er sich auch bei guter Prozeßstabilisierung mit Polymer belegt. Eine erneute Reinigung und Aktivierung wie z.B. durch Waschen mit Säure oder einem geeigneten Lösungsmittel zur Trocknung wie beispielsweise Aceton, führt nicht zum ursprünglichen Aktivitätsniveau zurück, so daß der Katalysator nach wenigen Einsatzzyklen ersetzt werden muß, will man nicht geringere Umsätze in Kauf nehmen.

Arbeitet man die Japanische Offenlegungsschrift JP-OS 54/126293 nach, erhält man nur 10 % Umsatz. Dieser geringe Umsatz bei der Reaktion ist bei den relativ teuren Ausgangsstoffen wirtschaftlich nicht sinnvoll. Bei einer sehr starken Inhibierung des Reaktionsgemisches erreicht man mit 75 %-iger Schwefelsäure zufriedenstellende Umsätze. Der zu treibende Aufwand bei der Inhibierung des Reaktionsgemisches hat aber im Produkt Nachteile, da es dann entsprechend schwer zu polymerisieren ist.

Eine weitere Alternative zur Synthese von Isobornyl(meth)acrylat ist die Umesterung von Methylmethacrylat ausgehend von Isoborneol. Das Verfahren der Umesterung von Methylmethacrylat zur Gewinnung von Estern der (Meth)acrylsäure ist bekannt.

### Umesterung

DE 196 02 035 (Röhm GmbH) beschreibt die Umesterung von (Meth)acrylsäureestern kurzkettiger Alkohole mit langkettigen Alkoholen (C₁ - C₂₈) zu (Meth)acrylsäureestern langkettiger Alkohole in Gegenwart eines Umesterungskatalysators aus Ca(OH)₂ oder eines Gemisches aus Ca(OH)₂ und LiCl.

Eine weitere Veröffentlichung (DE 19 54 709, Röhm GmbH) beschreibt ein katalytisches Verfahren zur Umesterung von (Meth)acrylsäureestern mit höchstens 250 ppm Ca(OH)₂ als Katalysator.

DE 195 45 870 (Röhm GmbH) offenbart eine Umesterung von niederen (Meth)acrylsäureestern zu (Meth)acrylsäureestern von Kohlehydraten mit mindestens einer freien Hydroxylgruppe. Als Umesterungskatalysator wird ein Gemisch aus Alkalicarbonat und einem quartären Ammoniumsalz eingesetzt.

DE 44 011 32 (Röhm GmbH) beschreibt die Umesterung von (Meth)acrylsäureestern mit Alkoholen, die eine oder mehrere veresterbare Hydroxylgruppen enthalten, in Gegenwart eines Mischkatalysators aus 5 - 95 Mol-% Diorganylzinnoxid und 95 - 5 Mol-% Diorganylzinndihalogeniden. Der Mischkatalysator wird in Mengen zwischen 0,01 - 10 Gew.-%, bezogen auf das Reaktionsgemisch eingesetzt.

DE 43 01 673 (Röhm GmbH) beschreibt den Einsatz einer Bleiverbindung als Umesterungskatalysator. Auch Dialkylzinnoxid alleine als Umesterungskatalysator wurde bereits beschrieben (DE 40 10 532, Röhm GmbH)

DE 31 46 191 (Röhm GmbH) beschreibt die Veresterung einer wässrigen (Meth)acrylsäure (Wassergehalt: 5 - 60 Gew.-%) mit einem Alkanol zu dem entsprechenden (Meth)acrylsäureester, in dem man die wässrige (Meth)acrylsäure in ein siedenden Veresterungsgemisch mit einem Gehalt an H₂SO₄ oder einer organischen Sulfonsäure leitet und den gebildeten Ester abdestilliert.

US-PS 3,087,962 (Rohm & Haas) beschreibt die Herstellung von Estern der Acrylsäure oder der Methacrylsäure durch Veresterung von Acrylsäure oder Methacrylsäure unter Katalyse von H₂SO₄ und BF₃.

Nach den bekannten Umesterungsvorschriften erhält man aus Isoborneol und (Meth)acrylsäuremethylester in glatter Reaktion Isobornylmethacrylat in hoher Reinheit und guter Ausbeute. Isoborneol ist aber kommerziell nicht verfügbar, nur Isobornylacetat, das auch als Riechstoff verwendet wird, ist im Handel wohlfeil erhältlich. Versuche zeigten aber, daß sich Isobornylacetat weder mit Methyl(meth)acrylat noch mit (Meth)acrylsäure zu Isobornyl(meth)acrylat in herkömmlicher Weise durch Zusatz eines bekannten Umesterungskatalysators umsetzen läßt.

### Aufgabe

Der Erfindung laß also die Aufgabe zugrunde, ausgehend von wohlfeilen Rohstoffen, eine industriell verwertbare Synthese für lsobornyl(meth)acrylat zu finden, die ohne den Einsatz komplizierter Mischkatalysatoren durchführbar ist und die keine übermäßige Stabilisierung des Reaktionsgemisches erfordert.

### Lösung

Es wurde nun gefunden, daß Isobornylacetat, das als Riechstoff verwendet wird und im Handel erhältlich ist, sich zu Isobornylmethacrylat umsetzen läßt. Dabei wurde weiter gefunden, daß sich allerdings das Isobornylacetat weder mit Methyl(meth)acrylat noch mit (Meth)acrylsäure zu Isobornyl(meth)acrylat in bekannter Weise durch Zusatz eines Umesterungskatalysators umsetzen läßt.

Es wurde gefunden, daß eine einfache Isobornyl(meth)acrylatsynthese dann erfolgreich ist, wenn man in einem Eintopfverfahren Isobornylacetat zunächst mit Methanol und einem Umesterungskatalysator zu Isoborneol und Methylacetat umsetzt und dann ohne Isolierung von Zwischenprodukten mit dem gleichen Katalysator wie in Stufe 1 unter Zusatz von Methyl(meth)acrylat daraus Isobornyl(meth)acrylat erzeugt. Der Rohester fällt schon dabei in sehr reiner Form an, so daß sich für einige Zwecke eine destillative Reinigung erübrigt. Selbstverständlich kann sich ein zusätzlicher Reinigungsschritt noch anschließen.

Als Umesterungskatalysatoren können beispielsweise verwendet werden: Bleiverbindungen, Zinkverbidnungen, Erdalkalimetalloxide oder Erdalkalimetallhydroxyde, wie beispielsweise CaO, Ca(OH)₂, MgO, Mg(OH)₂ oder Gemische aus den vorstehend genannten Verbindungen, ferner Alkalimetallhydroxide, -alkoxide und LiCl, es können weitere Gemische aus den vorstehend genannten Erdalkaliverbindungen und den Li-Salzen eingesetzt werden. Besonders bevorzugt ist ein Gemisch aus 75 Gew.% Calciumoxid und 25 Gew.% Lithiumhydroxid.

Die Menge an Katalysator oder Katalysatorgemische liegt zwischen 0,01 Gew.-% und 10 Gew.-%, bezogen auf das Reaktionsgemisch am Anfang der Reaktion.
Bevorzugt ist eine Menge von 0,5 Gew.-% bis 5 Gew.-%, besonders bevorzugt eine Menge von 1 Gew.-% bis 3 Gew.-%.

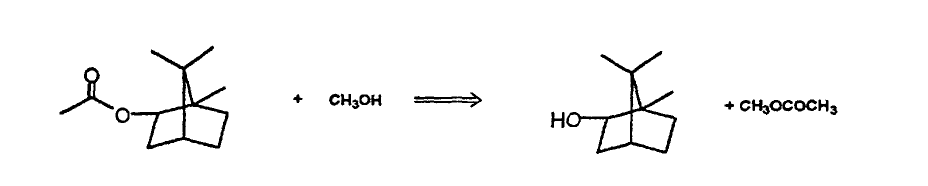

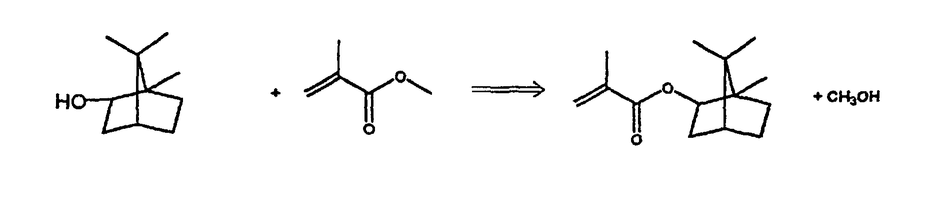

### Beispiele

### Beispiel 1

In einem 500 ml Vierhalskolben mit mechanischem Rührer, Innenthermometer, 30 cm Füllkörperkolonne (enthält Raschigringe) mit automatischem Kolonnenkopf und Gaseinleitrohr sowie Ölbadheizung legt man 196 g (1 mol) Isobornylacetat vor, gibt dann 480 g (15 mol) Methanol zu sowie als Katalysatoren 4,7 g Calciumoxid und 2 g Lithiumhydroxid (insgesamt 1 % bezogen auf die Gesamteinwaage). Man erhitzt unter Stickstoffeinleitung zum Sieden und zieht über den Kolonnenkopf in einem Kopftemperaturbereich von 57 bis 65 °C ein Gemisch aus Methylacetat und Methanol ab (Dauer 7 h). Danach wird restliches Methanol abdestilliert und anschließend 500 g (5 mol) Methyl(meth)acrylat, 0,033 g Hydrochinonmonomethylether und 0,002 g 4-Hydroxy-2,2,6,6-tetramethylpiperidyl-N-oxyl zugesetzt. Statt Stickstoff wird nun Luft eingeleitet und erneut zum Sieden erhitzt. Das entstehende Methanol-MMA-Azetrop wird von 64 bis 100 °C (Kopftemperatur) in 6 h abgezogen. Dann läßt man abkühlen, entfernt restliches MMA im Rotationsverdampfer und trennt den Katalysator durch Druckfiltration ab. Man erhält 179 g (81 % d. Th.) lsobornyl(meth)acrylat mit einer gaschromatographisch bestimmten Reinheit mit 94,7 Vol.-%.

### Beispiel 2

Durchführung wie in Beispiel 1, aber ohne N₂-Einleitung Unter Verwendung eines 2 l-Reaktionsgefäßes werden 521 g (2,5 mol) Isobornylacetat, 600 g (18 mol) Methanol, 3,36 g LiOH, 7,85 g CaO, 1,25 kg (12,5 mol) MMA, 0,083 g Hydrochinonmonomethylether und 0,006 g 4-Hydroxy-2,2,6,6-tetramethylpiperidyl-N-oxyl umgesetzt. Man erhält 443 g (80 % d. Th.) Isobornyl(meth)acrylat mit einer gaschromatographisch bestimmten Reinheit von 96,7 %. Das Produkt wird anschließend unter Zusatz von 20 ppm 4-Hydroxy-2,2,6,6-tetramethylpiperidyl-N-oxyl und 500 ppm Octadecyl-3-(3,5-di-tert-butyl-4-hydroxyphenyl)-propionat bei 10 mbar Druck über eine 20 cm Vigreuxkolonne fraktioniert. Man erhält 390 g Isobornyl(meth)acrylat, das eine Reinheit von 98 % aufweist.

### Vergleichsbeispiel 3

### Herkömmliche Umesterung aus Isobornylacetat, die nicht zum (Meth)acrylat führt

In der unter Beispiel 1 angegebenen Apparatur (1 l-Reaktionsgefäß, Rückflußkühler statt Kolonne) legt man 196 g (1 mol) Isobornylacetat und 344 g (4 mol) (Meth)acrylsäure vor. Man gibt 0,044 g Hydrochinonmonomethylether, 0,222 g Octadecyl-3-(3,5-di-tert-butyl-4-hydroxyphenyl)-propionat und 0, 004 g 4-Hydroxy-2,2,6,6-tetramethylpiperidyl-N-oxyl als Inhibitoren zu. Als Katalysatoren werden 2,5 g Dioctylzinnoxid und 2,9 g Dioctylzinndichlorid hinzugefügt. Unter Lufteinleitung erhitzt man 6 h auf 140 °C. Danach wird vom Kolbeninhalt ein Gaschromatogramm aufgenommen. Es ist kein Isobornyl(meth)acrylat nachweisbar.

### Vergleichsbeispiel 4

### Herkömmliche Umesterung aus Isobornylacetat, die nicht zum (Meth)acrylat führt

Das obige Vergleichsbeispiel wird mit 400 g Methyl(meth)acrylat an Stelle der (Meth)acrylsäure durchgeführt (Katalysatoren: 2,8 g Dioctylzinnoxid und 3,2 Dioctylzinndichlorid). Der Ansatz wird unter Lufteinleiten 6 h bei 90 °C gerührt. Es bildet sich kein Methylacetat, es findet also keine Umesterung statt.

### Vergleichsbeispiel 5

### Herkömmliche Umesterung aus Isobornylacetat, die nicht zum (Meth)acrylat führt

Das Beispiel 4 wird wiederholt, allerdings wird statt des Rückflußkühlers die Kolonne aus Beispiel 1 verwendet und als Katalysator 6 g Natriummethanolat eingesetzt. Man erhitzt zum Sieden und destilliert über den Kolonnenkopf MMA ab, das per GC auf Methylacetat geprüft wird. Trotz Nachdosierung von 50 g Methanol (nach 5 h Reaktionszeit) und 3 g Natriummethanolat (nach 8 h) läßt sich innerhalb von 14 h Reaktionszeit kein Methylacetat nachweisen.

## Patentansprüche

1. Eintopfverfahren zur Herstellung von Isobornyl(meth)acrylat,
**dadurch gekennzeichnet,**
**daß** man in einem ersten Verfahrensschritt Isobornylacetat mit Methanol als hydroxylgruppenhaltiger Verbindung mit einem Umesterungskatalysator zu Isoborneol umsetzt und dann ohne Aufarbeitung des Reaktionsgemisches das Isoborneol mit einem (Meth)acrylsäureester umsetzt.

2. Verfahren nach Anspruch 1,
**dadurch gekennzeichnet,**
**daß** man als Methylmethacrylsäureester Methylmethacrylat einsetzt.

3. Verfahren nach Anspruch 2,
**dadurch gekennzeichnet,**
**daß** das erhaltene Isobornylmethacrylat nicht mehr als 200 ppm (Gew.-ppm) an Stabilisator enthält.

## Claims

1. One-pot process for preparing isobornyl (meth)acrylate, **characterised in that** in a first step of the process isobornyl acetate is reacted with methanol as a compound containing hydroxyl groups together with a transesterification catalyst to obtain isoborneol and then without working up the reaction mixture the isoborneol is reacted with a (meth)acrylic acid ester.

2. Process according to claim 1, **characterised in that** methyl methacrylate is used as the methyl ester of methacrylic acid.

3. Process according to claim 2, **characterised in that** the isobornyl methacrylate obtained contains not more than 200 ppm (ppm by weight) of stabiliser.

## Revendications

1. Procédé en un seul pot pour la production de (méth)acrylate d'isobornyle,
**caractérisé en ce qu'**
en une seule étape de procédé l'acétate d'isobornyle avec le méthanol comme composé contenant un groupe hydroxyle et avec un catalyseur de transestérification est converti en isobornéol et ensuite sans traitement du mélange réactionnel on fait réagir l'isobornéol avec un ester d'acide (méth)acrylique.

2. Procédé selon la revendication 1,
**caractérisé en ce que**
comme ester d'acide (méth)acrylique le méthacrylate on met en oeuvre de méthyle.

3. Procédé selon la revendication 2,
**caractérisé en ce que**
le méthacrylate d'isobornyle obtenu ne contient pas plus de 200 ppm (ppm en poids) d'agent stabilisant.
